# FASCICULE DE BREVET EUROPEEN

(11) **EP 4 264 048 B1**
(45) Date de publication et mention de la délivrance du brevet: **05.02.2025**
(21) Numéro de dépôt: 21839954.1
(22) Date de dépôt: 16.12.2021
(51) Int. Cl.: F04B 9/04, F04B 13/00

(54) **DISPOSITIF DE POMPE AMÉLIORÉ**
VERBESSERTE PUMPVORRICHTUNG
IMPROVED PUMP DEVICE

(30) Priorité: 21.12.2020 FR 2013783
(43) Date de publication de la demande: 25.10.2023
(73) Titulaire: Abousaleh, Khaled, 8834 Schindellegi (CH)
(72) Inventeur: Abousaleh, Khaled, 8834 Schindellegi (CH)
(74) Mandataire: P&TS SA (AG, Ltd.)
(86) Numéro de dépôt international: PCT/EP2021/086110
(87) Numéro de publication internationale: WO 2022/136089

(56) Documents cités:
- WO-A1-03/073108
- FR-A1- 2 841 653
- US-A1- 2019 234 399

## Description

La présente invention se rapporte au domaine des pompes à seringues, notamment des pompes de précisions destinées à doser de petits volume: Voir US2019/234399 A1, qui divulgue un système selon l'état de la technique.

Un dosage précis, notamment dans le cas de médicaments, peut être vital. Or, les pompes peuvent perdre de leur précision en vieillissant. Il peut y avoir des fuites, par exemple au niveau des joints du piston de la seringue ou du raccordement de la seringue avec une tuyauterie d'arrivé ou de départ de fluides. Des jeux peuvent apparaitre ou s'aggraver entre différents organes de la pompe. Des blocages ou des obstacles peuvent aussi empêcher un fonctionnement normal de la pompe.

Le but de l'invention est de proposer une pompe équipée de moyens pour surveiller son fonctionnement, et garantir un dosage plus précis que dans les pompes de l'art antérieur.

Selon un premier objet de l'invention, un système pour actionner un piston d'une seringue dans une pompe à seringue, le piston étant muni d'un connecteur, comprend :
- un moteur ;
- des moyens de transmission ; et,
- un poussoir ;

les moyens de transmission étant prévus pour transmettre une action du moteur au poussoir et le poussoir étant prévu pour déplacer le piston en translation selon un axe de seringue,
le poussoir comprenant :
   - une prise pour le connecteur ;
   - un segment de fixation prévu pour être fixé aux moyens de transmission ;
   - une poutre qui relie la prise au segment de fixation, transversalement à l'axe de seringue ; et,
   - un capteur pour mesurer une flexion de la poutre autour d'un axe perpendiculaire à l'axe de seringue.

Le moteur est de préférence un moteur rotatif dont un arbre forme une vis, les moyens de transmission comprenant une noix vissée sur la vis et une potence liée en translation avec cette noix, le segment de fixation du poussoir étant fixé à la potence. Le moteur est avantageusement un moteur pas à pas.

Le capteur peut être une jauge de contrainte collée sur une face de la poutre.

Avantageusement, la prise et le connecteur forment une liaison linéaire-annulaire entre eux. Le connecteur peut comprendre une sphère formée à une extrémité d'une tige axiale du piston et la prise peut comprendre une gorge cylindrique et une fente, la gorge étant prévue pour y loger la sphère, s'étendant selon un axe sensiblement perpendiculaire à l'axe de seringue et ayant un diamètre sensiblement égal à un diamètre de la sphère, et, la fente étant prévue pour le passage avec jeu de la tige lorsque la sphère est dans la gorge. La gorge peut comprendre une ouverture axiale pour y introduire le connecteur.

Selon un deuxième objet de l'invention, une pompe de précision comprend un système selon le premier objet de l'invention. Elle comprend de préférence, des moyens de prise pour y monter de façon amovible une seringue selon le deuxième objet de l'invention.

Une telle pompe comprend avantageusement au moins un capteur parmi un capteur de courant pour le moteur, un capteur de pression de l'air ambiant, un capteur de température ambiante, un capteur de vibrations, un capteur d'infrarouges, un capteur d'ultrasons et un capteur d'humidité. Elle peut aussi comprendre des moyens contrôle à distance et des moyens pour transmettre des données relevées par le capteur de flexion et, le cas échéant, un ou plusieurs autres capteurs aux moyens de contrôle à distance.

Selon un troisième objet de l'invention, un procédé pour contrôler une usure d'une seringue dans une pompe selon le troisième objet de l'invention, utilise des données relevées par le capteur de flexion et, le cas échéant, un ou plusieurs autres capteurs. De préférence, ce procédé comprend une étape préalable d'apprentissage.

Plusieurs modes d'exécution de l'invention seront décrits ci-après, à titre d'exemples non limitatifs, en référence aux dessins annexés dans lesquels :
[Fig. 1] est une vue schématique en perspective, de trois-quarts avant gauche, d'une pompe à seringue selon l'invention ;

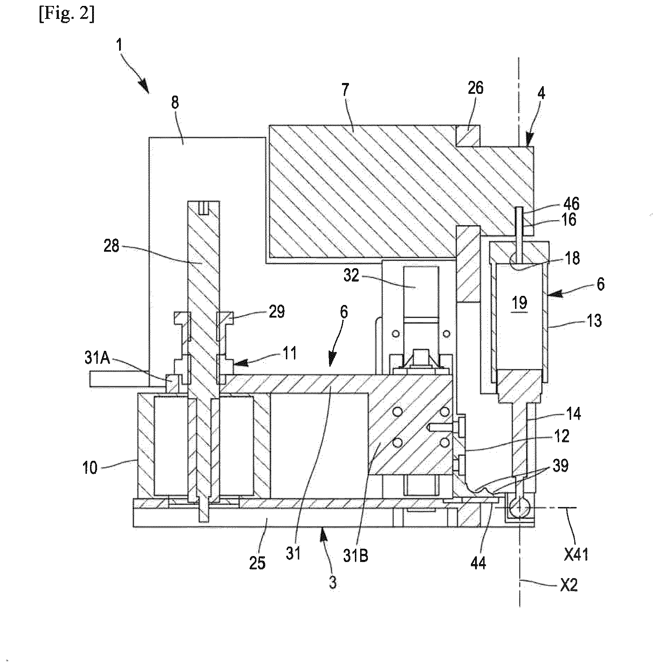

[Fig. 2] est une vue schématique en coupe longitudinale et en élévation de la pompe de la figure 1 ;
[Fig. 3] est une vue schématique en élévation longitudinale de la partie avant de la pompe de la figure 1, au voisinage de la seringue ;
[Fig. 4] est une vue schématique en perspective, de trois-quarts avant droit, d'un poussoir pour la seringue de la figure 3 ;
[Fig. 5] est une vue schématique, en élévation et de face, d'un détail de moyens d'actionnement de la seringue ; et,
[Fig. 6] est une vue schématique en perspective, de trois-quarts avant droit, similaire à celle de la figure 4, illustrant un autre mode de réalisation d'un poussoir utilisable dans la pompe de la figure 1.

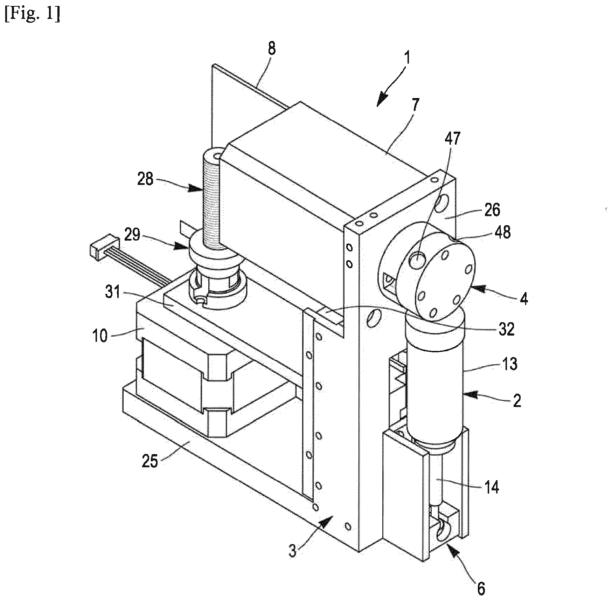

La figure 1 représente une pompe de précision 1 selon l'invention. Cette pompe comprend et utilise une seringue 2. Elle comprend en outre :
- un châssis 3 ;
- une vanne 4 ;
- des moyens 6 pour actionner la seringue ;
- un moteur de vanne 7 pour actionner la vanne 4 ; et,
- des moyens de contrôle électroniques 8.

Les moyens d'actionnement 6 de la seringue comprennent un moteur de seringue 10, des moyens de transmission 11 et un poussoir 12.

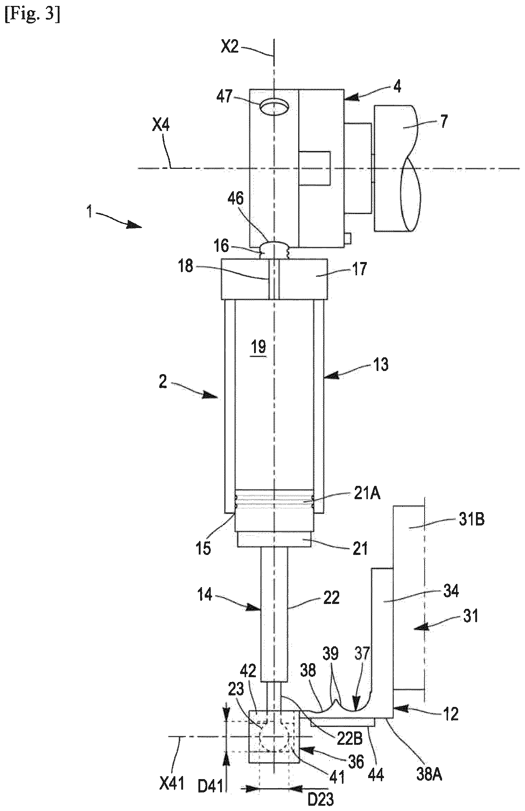

La seringue est sensiblement de révolution autour d'un axe de seringue X2. Dans l'exemple illustré, l'axe de seringue est vertical. Comme particulièrement illustré à la figure 3, la seringue 2 comprend un cylindre 13, un piston 14 mobile au travers d'une ouverture axiale 15 du cylindre, et une prise fluidique axiale 16. La prise fluidique 16 est une prise mâle, formée à l'extérieur du cylindre, à l'opposé de l'ouverture 15 dans un fond 17 du cylindre. La seringue comprend en outre un canal axial 18 qui permet une circulation d'un fluide entre une chambre 19 du cylindre et l'extérieur de la seringue, au travers du fond 17 et de la prise fluidique 16. La chambre 19 est définie entre le cylindre et le piston ; son volume intérieur varie en fonction de la position du piston dans le cylindre.

Dans l'exemple illustré, le piston 14 comprend une tête 21, montée pour coulisser de façon étanche dans le cylindre 13, une tige 22 qui s'étend axialement depuis la tête à l'extérieur du cylindre, et, un connecteur 23 formé à une extrémité de la tige opposée à la tête 21. La tête comprend un joint annulaire 21A, pour faire une étanchéité entre le piston14 et le cylindre 13. Dans l'exemple illustré, le connecteur 23 à la forme d'une sphère 23 montée à une partie 22B de section réduite de la tige 22. La tige et sa partie de section réduite ont chacune une section circulaire, autour de l'axe de seringue X2. La partie de section réduite a un diamètre constant noté D22B. La sphère 23 a un diamètre noté D23.

La vanne 4 est une vanne rotative autour d'un axe de vanne X4. Elle est actionnée par le moteur de vanne 7. Elle comprend trois ports 46-48. Dans l'exemple illustré, les trois ports sont des prises fluidiques femelles. Une première prise fluidique femelle 46 est prévue pour venir en prise avec la prise fluidique mâle 16 de la seringue. Elle comprend en outre une deuxième prise fluidique femelle 47 et une troisième prise fluidique femelle 48, chacune pour y brancher un tuyau. Par exemple un tuyau peut être relié à une réserve pour y pomper le fluide, et un autre tuyau pour verser un volume voulu de fluide ainsi pompé. Une rotation de la vanne permet de faire communiquer la première prise 46, soit avec la deuxième prise 47, soit avec la troisième prise 48.

Le châssis 3 est réalisé par injection, en aluminium. Il a la forme d'un « L ». Il comprend une base 25 en forme de plaque sensiblement rectangulaire et allongée d'avant en arrière. Il comprend aussi un montant sensiblement vertical 26 qui s'étend vers le haut à partir d'une extrémité avant de la base 25.

Le moteur 10 de la seringue est fixé à une extrémité arrière de la base 24. Il comprend un arbre 28 disposé verticalement et formant une vis. Les moyens de transmission 11 comprennent une noix d'entrainement 29 et une potence 31. La noix 29 est en prise avec la vis 28. La potence s'étend d'arrière en avant ; elle est en prise par une extrémité arrière 31A avec la noix 29 et en prise avec un guide 32 linéaire par une extrémité avant 31B. Le guide 32 est fixé au montant 26 du châssis 3. La noix entraine verticalement la potence 31 et le guide 32 maintient la potence en translation verticale.

Un codeur linéaire est fixé au châssis 3, et une règle graduée est fixée à la potence 31, en vis-à-vis du codeur linéaire. Le mouvement de la règle graduée est lu par un codeur linéaire. Le couple codeur linéaire-règle permet de vérifier avec une grande précision qu'un mouvement a lieu et permet de déterminer une position sensiblement absolue du piston relativement au châssis.

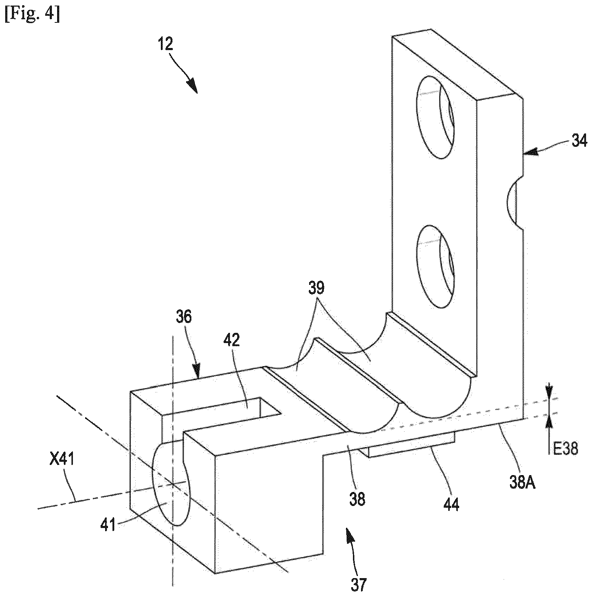

Le poussoir 12 est fixé à l'extrémité avant 31B de la potence 31. Comme particulièrement illustré à la figure 4, le poussoir 12 a une forme d'un « L », dont le montant est référencé 34 et dont la base est référencée 37. Le montant 34 du « L » sert de segment pour fixer le poussoir à la potence ; il est plaqué contre une face avant verticale de l'extrémité avant 31B de la potence 31 ; le montant 34 y est fixé par vissage. La base 37 s'étend sensiblement horizontalement, vers l'avant, depuis une extrémité inférieure du montant 34. La base 37 du poussoir comprend une prise 36 et une poutre 38 qui relie horizontalement la prise à une extrémité inférieure du montant 34 du poussoir 12.

La prise 36 du poussoir 12 comprend un canal cylindrique 41 autour d'un axe de prise X41 sensiblement horizontal. Le canal est ouvert sur toute sa section au travers d'une face avant de la prise. Il est aussi ouvert dans une face supérieure de la prise, une fente reliant le canal avec cette face supérieure. Le canal 41 a un diamètre D41 sensiblement égal au diamètre D23 de la sphère 23, de sorte que la sphère forme une liaison linéaire-annulaire avec le canal et peut y coulisser, selon l'axe de prise X41. La fente 42 a une largeur L42 supérieure au diamètre D22B de la section réduite 22B de la tige 22 de la seringue 6, de sorte qu'un jeu est ménagé de part et d'autre de la partie 22B de la tige, de façon à autoriser un basculement de la tige autour de de l'axe de prise X41.

La seringue 2 est démontable c'est-à-dire que l'on peut remplacer une seringue par une autre. Pour mettre en place une seringue, il convient de mettre en vis-à-vis la prise fluidique mâle 16 et la première prise fluidique femelle 46, d'insérer le connecteur 23, ici la sphère 23, dans la prise 36, de sorte que l'axe X2 de la seringue soit sensiblement vertical, puis, de visser entre elles les prises fluidiques 16, 46.

Le poussoir permet, par l'action du canal sur la sphère, de pousser du bas vers le haut, et de tirer du haut vers le bas, le piston 14. La liberté de mouvement de la sphère 23 dans le canal 41 garantit que l'action du poussoir sur la tige est dirigée selon l'axe X2 de la seringue.

La poutre 38 comprend une surface inférieure sensiblement horizontale 38A. Une jauge de contrainte 44 est fixée à la face inférieure 38A. Cette jauge est prévue pour mesurer des contraintes de flexion autour d'un axe horizontal transversal à la poutre. Les contraintes ainsi mesurées sont essentiellement dépendantes des efforts exercés par la seringue 2 sur le poussoir 12.

Lorsque le poussoir 12 se déplace et déplace avec lui le piston 14 de la seringue 2, des efforts sont générés sur le poussoir. Ces efforts proviennent des forces de frottement du joint 21A du piston et de la pression du fluide se trouvant dans la chambre 19 de la seringue. La jauge 44 permet notamment d'évaluer les forces de frottement du j oint et l'usure du joint.

Des zones d'affaiblissement 39 sont formées dans la partie supérieure de la poutre 38. Elles permettent d'augmenter la sensibilité de la jauge 44. Elles sont disposées de façon à conserver une raideur suffisante à la poutre 38 pour garantir une précision satisfaisante du déplacement du piston 14, donc du dosage. Dans l'exemple illustré, les zones d'affaiblissement sont constituées de deux demi-cylindres 39, transversaux à la poutre, ouverts vers le haut dans une face supérieure de la poutre. Une épaisseur courante de la poutre, mesurée entre la face inférieure et le fond des zones d'affaiblissement 39, est notée E38. C'est cette épaisseur qui détermine l'intervalle de forces que la jauge 44 est capable de mesurer.

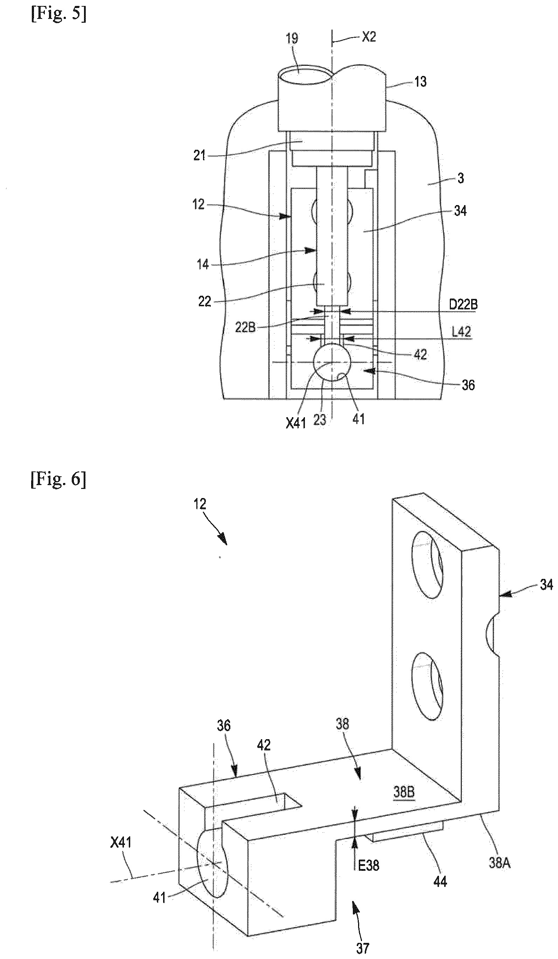

La figure 6 illustre un deuxième mode de réalisation pour le poussoir 12. Il diffère du premier mode de réalisation, illustré à la figure 4, en ce que la section de la poutre 38, transversalement à l'axe de prise X41 est sensiblement constante et rectangulaire. Dans cette configuration, si elle permet d'avoir des contraintes uniformes dans la poutre, pour une même épaisseur courante E8 de la poutre, le déplacement en flexion de la poutre est supérieur au même déplacement, dans le cas du premier mode de réalisation.

Dans ce deuxième mode de réalisation, la jauge 44 peut être fixée sur une face supérieure 38B de la poutre 38, au lieu d'être disposée sur la face inférieure 38A. Aussi, on peut utiliser deux jauges, l'une sur la face inférieure 38A, l'autre sur la face supérieure 38B.

Mesurer les forces de frottement du joint en temps réel permet de suivre l'évolution de l'usure du joint dans le temps et permet de prévenir des fuites et d'anticiper le remplacement du joint, ou celui de la seringue dans son ensemble.

Dans le cas où un système selon l'invention est utilisé pour effectuer un dosage, dans le cadre d'un diagnostic, les risques d'erreurs sont réduits, ce qui est bénéfique pour le patient concerné.

La mesure d'usure du joint est très importante car elle permet d'éviter les fuites et donc les erreurs de dosage qui en découlent. Lorsque la pompe est une pompe de médicament reliée à une perfusion, cela diminue les risques pour un patient ainsi perfusé.

Mesurer cette usure permet aussi de planifier le remplacement de la seringue et de le faire au bon moment, c'est-à-dire ni trop tôt, ni trop tard. Le remplacement est ainsi basé sur une mesure physique.

En outre, la pompe 1 comprend des moyens de commande pour chacun des moteurs

Toute variation de pression générée dans la chambre se transforme en force et est mesurée par la jauge de contrainte.

Avec un seul capteur, ici la jauge 44, on peut mesurer à la fois une usure et une pression.

Le moteur 10 qui actionne le poussoir 12 et le moteur 7 qui actionne la vanne 4 sont des moteurs pas à pas. Les moyens de commande 8 comprennent des moyens de commande pour les moteurs 7, 10 comprenant, pour chaque moteur 7, 10, un capteur de courant. Chaque capteur de courant permet de réaliser les fonctions suivantes :
- Contrôle de l'échauffement du moteur, qui résulte du courant circulant dans le moteur ;
- Contrôle du couple du moteur, notamment du moteur 10 de la seringue, puisqu'il y a un lien entre courant et couple ;
- Prédiction de l'usure du moteur, un lien existant entre le courant consommé et l'usure de la pompe. Un suivi à distance est avantageusement mis en oeuvre. Une application comprenant un algorithme, basé sur de l'intelligence artificielle, peut avantageusement être prévue pour aider au suivi à distance.

La pompe comprend aussi un capteur qui permet de suivre sa consommation électrique globale ce qui, outre un contrôle de la consommation globale, permet de prédire une durée de vie globale ou restante pour la pompe 1.

La pompe peut avantageusement comprendre d'autres capteurs, notamment choisis parmi un capteur de pression de l'air ambiant, un capteur de température ambiante, un capteur de vibrations, un capteur d'infrarouges, un capteur d'ultrason et/ou un capteur d'humidité.

Il y a un lien entre la pression de l'air et la fonction de dosage de la pompe. En effet, la fonction de dosage est une relation entre la variation de pression générée par le mouvement du piston et la pression atmosphérique présente au bout du tuyau (ou aiguille de prélèvement). Donc, le capteur de pression de l'air ambiant, c'est-à-dire celui d'un lieu où se trouve la pompe, permet de corriger le volume généré par la fonction de dosage.

Il y a aussi un lien entre volume et température. Grâce au capteur de température ambiante, la fonction dosage est corrigée ou ajustée en fonction de la température ambiante. Ce capteur de température permet aussi de s'assurer que le produit fonctionne dans l'intervalle de température spécifié.

La pompe 1 comprend plusieurs mouvements mécaniques qui génèrent des vibrations. Le capteur de vibration permet de suivre l'évolution des niveaux de vibration afin de prédire et anticiper des pannes mécaniques ainsi que les dis fonctionnements.

Le capteur ultrason a pour objectif de mesurer un son généré par les mouvements mécaniques, aussi afin de prédire et/ou anticiper des pannes mécaniques ainsi que les disfonctionnements.

Un capteur infrarouge est avantageusement dirigé vers chacun des moteurs 7, 10. Il permet de mesurer sans contact la température réelle du moteur. Étant donné que la pompe 1 est généralement installée dans un milieu confiné, par exemple à l'intérieur d'une machine d'analyse, un contrôle précis des températures des moteurs et de l'échauffement en général est avantageux.

Le capteur d'humidité permet de mesurer l'humidité ambiante. Il permet de s'assurer que la pompe fonctionne dans un intervalle d'humidité spécifié. En outre, en cas de fuite de la pompe, le taux d'humidité augmente et le capteur d'humidité peut ainsi permettre de détecter une telle fuite.

Les moyens de contrôle 8 de la pompe 1 comprennent avantageusement des moyens de liaison vers un dispositif de contrôle distant et/ou par exemple vers un « cloud ». Ces moyens de liaison peuvent comprendre une connexion cellulaire, Bluetooth et/ou Wi-Fi. Ces moyens de liaison permettent d'envoyer des données ou des mesures recueillies par les capteurs aux moyens de contrôle distants, en temps réel ou en différé.

Les moyens de contrôle 8 de la pompe sont avantageusement prévus pour recevoir des informations entrantes. Ces informations entrantes peuvent être des configurations spécifiques ou une mise à jour d'un logiciel.

L'envoi de données des capteurs sur le cloud afin de les analyser en temps réel ou en décalé permet de fournir à l'utilisateur des services importants comme :
- une prédiction d'usure et anticipation de remplacement de la seringue ;
- une prédiction d'un besoin de maintenance ;
- une prédiction de panne ;
- une prédiction de fin de vie ; et/ou,
- une fourniture d'alarmes et d'informations lorsque l'utilisation du produit sort des spécifications, par exemple d'un intervalle de températures ou d'hygrométrie spécifique, afin de garantir que les résultats de dosage sont conformes aux attentes d'un utilisateur.

Une pompe à seringue selon l'invention a les caractéristiques suivantes :
- le mouvement linéaire du piston 14 est réalisé grâce à une liaison mécanique entre une vis 28 et une noix 29 alors que les pompes de l'art antérieur utilisent une courroie. La liaison vis/noix apporte les améliorations suivantes :
   - simplicité mécanique qui réduit le nombre de pièces ;
   - réduction des jeux mécaniques totaux, ce qui permet d'améliorer la précision de la pompe ;
   - gain de place, du fait d'un nombre de pièce réduit ;
   - des vitesses et accélérations plus élevées sont possibles ;
   - fabrication simplifiée car ne nécessitant pas de réglage de la tension d'une courroie ; et,
   - durée de vie plus élevée que la durée de vie d'une courroie.
- le mouvement linéaire du piston 14 est en outre assuré par le guide linéaire 32 afin de supporter les efforts ;
- le poussoir 12 a pour fonction de pousser et tirer le piston 14 de la seringue 2 afin de réaliser la fonction de dosage.

Une même pompe selon l'invention peut être prévue pour accepter plusieurs tailles de seringue, par exemple des seringues pouvant contenir 100µl, 250µl, 500µl, 1000µl, 2,5ml, 5ml, 12,5µl, 25ml ou 50ml. Une telle pompe peut être prévue pour doser avec précision des volumes compris entre environ 0,1µl et 50ml.

Bien sûr, l'invention n'est pas limitée aux exemples qui viennent d'être décrits. Au contraire, l'invention est définie par les revendications qui suivent.

Il apparaîtra en effet à l'homme de l'art que diverses modifications peuvent être apportées aux modes de réalisation décrits ci-dessus, à la lumière de l'enseignement qui vient de lui être divulgué.

Notamment, l'axe de seringue peut ne pas être vertical. La pompe peut aussi comprendre plusieurs seringues, entrainées par un même poussoir ou pas.

De plus, au lieu d'être en aluminium, le châssis peut aussi être en un matériau plastique.

## Revendications

1. Système (6) pour actionner un piston (14) d'une seringue (2) dans une pompe (1) à seringue, le piston étant muni d'un connecteur (23), comprenant
- un moteur (10) ;
- des moyens de transmission (11) ; et,
- un poussoir (12) ;
les moyens de transmission étant prévus pour transmettre une action du moteur au poussoir et le poussoir étant prévu pour déplacer ledit piston en translation selon un axe de seringue (X2),
ledit poussoir comprenant :
- une prise (36) pour ledit connecteur (23) ;
- un segment de fixation (34) prévu pour être fixé auxdits moyens de transmission ;
- une poutre (38) qui relie la prise (36) au segment de fixation, transversalement à l'axe de seringue (X2) ; **caractérisé en ce qu'**il comprend:
- un capteur (44) pour mesurer une flexion de ladite poutre (38) autour d'un axe perpendiculaire audit axe de seringue (X2).

2. Système selon la revendication 1, **caractérisé en ce que** le moteur (10) est un moteur rotatif comprenant un arbre formant une vis (28) et **en ce que** les moyens de transmission comprennent une noix (29) vissée sur la vis (28) et une potence (31) liée en translation avec ladite noix, le segment de fixation (34) du poussoir (12) étant fixé à ladite potence (31).

3. Système selon l'une des revendications 1 et 2, **caractérisé en ce que** le moteur (10) est un moteur pas à pas.

4. Système selon l'une des revendications 1 à 3, **caractérisé en ce que** le capteur (44) est une jauge de contrainte collée sur une face (38A) de la poutre (38).

5. Système selon l'une des revendications 1 à 4, **caractérisé en ce que** la prise (36) et le connecteur (23) forment une liaison linéaire-annulaire entre eux.

6. Système selon la revendication 5, **caractérisé en ce que** le connecteur comprend une sphère (23) formée à une extrémité d'une tige axiale (22B) du piston (14) et **en ce que** la prise (36) comprend une gorge cylindrique (41) et une fente (42), ladite gorge (41) étant prévue pour y loger ladite sphère, s'étendant selon un axe (X41) sensiblement perpendiculaire à l'axe de seringue (X2) et ayant un diamètre (D41) sensiblement égal à un diamètre (D23) de ladite sphère, et, ladite fente (42) étant prévue pour le passage avec jeu de ladite tige (22B) lorsque la sphère est dans ladite gorge.

7. Système selon la revendication 6, **caractérisé en ce que** la gorge comprend une ouverture axiale pour y introduire le connecteur.

8. Pompe de précision (1), caractérisée en ce quelle comprend un système selon l'une des revendications 1 à 7.

9. Pompe selon la revendication 8, **caractérisée en ce qu'**elle comprend des moyens de prise (36, 46) pour y monter de façon amovible une seringue.

10. Pompe selon l'une des revendications 8 et 9, **caractérisé en ce qu'**elle comprend en outre au moins un capteur parmi un capteur de courant pour le moteur (10), un capteur de pression de l'air ambiant, un capteur de température ambiante, un capteur de vibrations, un capteur d'infrarouges, un capteur d'ultrasons et un capteur d'humidité.

11. Pompe selon l'une des revendications 11 à 13, **caractérisée en ce qu'**elle comprend des moyens de contrôle à distance et des moyens pour transmettre des données relevées par le capteur de flexion (44) et, le cas échéant, un ou plusieurs autres capteurs auxdits moyens de contrôle à distance.

12. Procédé pour contrôler une usure d'une seringue dans une pompe selon l'une des revendications 8 à 11, **caractérisé en ce qu'**il utilise des données relevées par le capteur de flexion (44) et, le cas échéant, un ou plusieurs autres capteurs.

13. Procédé selon la revendication 12, **caractérisé en ce qu'**il comprend une étape préalable d'apprentissage.

## Patentansprüche

1. System (6) zum Betätigen eines Kolbens (14) einer Spritze (2) in einer Spritzenpumpe (1), wobei der Kolben mit einem Anschluss (23) versehen ist, umfassend:
- einen Motor (10);
- Übertragungsmittel (11); und
- ein Drücker (12);
wobei die Übertragungsmittel dazu vorgesehen sind, um eine Aktion vom Motor auf den Drücker zu übertragen, und wobei der Drücker dazu vorgesehen ist, den besagten Kolben translatorisch entlang einer Spritzenachse (X2) zu bewegen,
wobei der besagte Drücker umfasst:
- eine Buchse (36) für den besagten Stecker (23);
- ein Befestigungssegment (34), welches dazu bestimmt ist, an den besagten Übertragungsmitteln befestigt zu werden;
- einen Balken (38), welcher die Buchse (36) quer zur Spritzenachse (X2) mit dem Befestigungssegment verbindet;
**dadurch gekennzeichnet, dass** es umfasst:
- einen Sensor (44) zum Messen einer Biegung des besagten Balkens (38) um eine Achse senkrecht zur besagten Spritzenachse (X2).

2. System gemäss Anspruch 1, **dadurch gekennzeichnet, dass** der Motor (10) ein Rotationsmotor ist, welcher eine Welle aufweist, die eine Schraube (28) bildet, und dass die Übertragungsmittel eine auf die Schraube (28) aufgeschraubte Mutter (29) und eine Trägereinrichtung (31), welche translatorisch mit der besagten Mutter verbunden ist, umfasst, wobei das Befestigungssegment (34) des Drückers (12) an der besagten Trägereinrichtung (31) befestigt ist.

3. System gemäss einem der Ansprüche 1 und 2, **dadurch gekennzeichnet, dass** der Motor (10) ein Schrittmotor ist.

4. System gemäss einem der Ansprüche 1 bis 3, **dadurch gekennzeichnet, dass** der Sensor (44) ein Dehnungsmessstreifen ist, welcher auf einer Seite (38 A) des Balkens (38) aufgeklebt ist.

5. System gemäss einem der Ansprüche 1 bis 4, **dadurch gekennzeichnet, dass** die Buchse (36) und der Stecker (23) zwischen sich eine linearringförmige Verbindung bilden.

6. System gemäss Anspruch 5, **dadurch gekennzeichnet, dass** der Stecker eine Kugel (23) umfasst, welche an einem Ende einer axialen Stange (22B) des Kolbens (14) ausgebildet ist, und dass die Buchse (36) eine zylindrische Nut (41) und einen Schlitz (42) umfasst, wobei die besagte Nut (41) zur Aufnahme der besagten Kugel vorgesehen ist, sich entlang einer Achse (X41) im Wesentlichen senkrecht zur Spritzenachse (X2) erstreckt und einen Durchmesser (D41) aufweist, der im Wesentlichen gleich einem Durchmesser (D23) der besagten Kugel ist, und wobei der besagte Schlitz (42) für den Durchgang mit Spiel der besagten Stange (22B) vorgesehen ist, wenn sich die Kugel in der besagten Nut befindet.

7. System gemäss Anspruch 6, **dadurch gekennzeichnet, dass** die Nut eine axiale Öffnung zum Einführen des Steckers aufweist.

8. Präzisionspumpe (1), **dadurch gekennzeichnet, dass** sie ein System gemäss einem der Ansprüche 1 bis 7 umfasst.

9. Pumpe gemäss Anspruch 8, **dadurch gekennzeichnet, dass** sie Greifmittel (36, 46) zur abnehmbaren Halterung einer Spritze umfasst.

10. Pumpe gemäss einem der Ansprüche 8 und 9, **dadurch gekennzeichnet, dass** sie zudem mindestens einen Sensor umfasst, ausgewählt aus einem Stromsensor für den Motor (10), einem Umgebungsluftdrucksensor, einem Umgebungstemperatursensor, einem Vibrationssensor, einem Infrarotsensor, einem Ultraschallsensor und einem Feuchtigkeitssensor.

11. Pumpe gemäss einem der Ansprüche 11 bis 13, **dadurch gekennzeichnet, dass** sie Fernsteuerungsmittel und Mittel zur Übertragung der vom Biegesensor (44) und gegebenenfalls von einem oder mehreren anderen Sensoren aufgezeichneten Daten an die besagten Fernsteuerungseinrichtung umfasst.

12. Verfahren zur Verschleissüberwachung einer Spritze in einer Pumpe gemäss einem der Ansprüche 8 bis 11, **dadurch gekennzeichnet, dass** es die vom Biegesensor (44) und gegebenenfalls von einem oder mehreren weiteren Sensoren erfassten Daten nutzt.

13. Verfahren gemäss Anspruch 12, **dadurch gekennzeichnet, dass** es einen vorangehenden Lernschritt umfasst.

## Claims

1. System (6) for actuating a piston (14) of a syringe (2) in a syringe pump (1), the piston being provided with a connector (23), comprising :
- a motor (10);
- transmission means (11); and
- a pusher (12);
the transmission means being designed to transmit an action of the motor to the pusher and the pusher being designed to move the piston in translation along a syringe axis (X2),
said pusher comprising:
- a socket (36) for said connector (23);
- an attachment segment (34) designed to be attached to said transmission means;
- a beam (38) that connects the socket (36) to the attachment segment, transversely to the syringe axis (X2);
**characterized in that** it comprises
- a sensor (44) for measuring a bending of said beam (44) about an axis perpendicular to the syringe axis (X2).

2. System according to claim 1, **characterized in that** the motor (10) is a rotary motor comprising a shaft forming a screw (28) and wherein the transmission means comprise a nut (29) screwed onto the screw (28) and a bracket (31) translationally connected with said nut, the attachment segment (34) of the pusher (12) being attached to said bracket (31).

3. System according to one of claims 1 and 2, **characterized in that** the motor (10) is a stepping motor.

4. System according to one of claims 1 to 3, **characterized in that** the sensor (44) is a strain gauge glued to a face (38A) of the beam (38).

5. System according to one of claims 1 to 4, **characterized in that** the socket (36) and the connector (23) form a linear-annular connection between them.

6. System according to claim 5, **characterized in that** the connector comprises a sphere (23) formed at one end of an axial rod (22B) of the piston (14) and **in that** the socket (36) comprises a cylindrical groove (41) and a slot (42), said groove (41) being designed to house said sphere therein, extending along an axis (X41) substantially perpendicular to the syringe axis (X2) and having a diameter (D41) substantially equal to a diameter (D23) of said sphere, and said slot (42) being provided for the passage with clearance of said rod (22B) when the sphere is in said groove.

7. System according to claim 6, **characterized in that** the groove comprises an axial opening for introducing the connector therein.

8. Precision pump (1), **characterized in that** it comprises a system according to one of claims 1 to 7.

9. Pump according to claim 8, **characterized in that** it comprises socket means (36, 46) for removably mounting a syringe therein.

10. Pump according to one of claims 8 and 9, **characterized in that** it further comprises at least one sensor from a current sensor for the motor (10), a sensor for the pressure of the ambient air, an ambient temperature sensor, a vibration sensor, an infrared sensor, an ultrasound sensor and a moisture sensor.

11. Pump according to one of claims 11 to 13, **characterized in that** it further comprises remote-control means and means for transmitting data read by the bending sensor (44) and, where applicable, one or more other sensors to said remote-control means.

12. Method for monitoring wear on a syringe in a pump according to one of claims 8 to 11, **characterized in that** the method uses data read by the bending sensor (44) and, where applicable, one or more other sensors.

13. Method according to claim 12, **characterized in that** it further comprises a prior learning step.
